# EUROPEAN PATENT APPLICATION

(11) **EP 3 729 996 A1**
(43) Date of publication of application: **28.10.2020**
(21) Application number: 18893171.1
(22) Date of filing: 21.05.2018
(51) Int. Cl.: A45D 44/00, A61K 8/02, A45D 44/22, A61Q 19/00, D04B 1/22, A61N 1/32, A61N 1/36, A61N 1/04

(54) **THIN FILM MASK PACK HAVING EXCELLENT WEAR SENSATION, ELECTRICAL CONDUCTIVITY AND ANTIBACTERIAL PROPERTY, AND MANUFACTURING METHOD THEREFOR**

(30) Priority: 22.12.2017 KR 20170177703; 23.02.2018 KR 20180021833; 23.02.2018 KR 20180021842
(71) Applicant: LSK Finetex Co.,Ltd., Seoul 07295 (KR)
(72) Inventor: KIM, Jang Whan, Goyang-si Gyeonggi-do 10385 (KR)
(74) Representative: Beck & Rössig European Patent Attorneys
(86) International application number: PCT/KR2018/005773
(87) International publication number: WO 2019/124651

(57) **Abstract**

Disclosed is a mask sheet and a sheet mask using the same, and more particularly an eco-friendly mask sheet having electrical conductivity and antibacterial function and a sheet mask using the same. The sheet mask includes a knit mask sheet having 1 to 20 wt.% of an electrically conductive fiber containing a copper component; and a cosmetic composition soaking into the mask sheet.

## Description

### TECHNICAL FIELD

The present invention relates to a mask sheet and a sheet mask using the same, and more particularly to an eco-friendly mask sheet having electrical conductivity and antibacterial function and a sheet mask using the same.

### BACKGROUND ART

In general, a sheet mask is a sheet of nonwoven fabric cut into the shape of a face and soaked in an intense serum containing a variety of functional ingredients beneficial to the skin. Placing the sheet mask on the face for a defined period of time allows the functional ingredients in the serum to penetrate into the skin of the face for facial skincare. The sheet mask using a sheet of nonwoven fabric has a low price but fails to create a tight seal to the face. On account of this, hydrogel or bio-cellulose sheets that ensure a tight seal are being developed.

There have recently been devised whole new methods for providing various functional properties by making a conductive mask sheet and applying a voltage to the conductive mask sheet to enhance the absorbency of the mask sheet for the beneficial ingredients infiltrated in the sheet mask. KR Patent Laid-Open Publication No. 10-2016-0035205 filed by Cheul Park, for example, discloses a method of adhering a conductive sheet to a mask sheet to promote the absorbency of the mask sheet for beneficial ingredients. In addition, Korean Patent No. 1689345 granted to Jeong-Ho Cho specifies a method of using a smartphone current to apply stimulation to a sheet mask having a patterned conductive carbon film support. Yet, due to the use of conductive materials, these conventional conductive mask sheets feature a rigid feel and poor tactile quality and fail to form a tight seal against the skin, so they are unable to get more marketable despite their usefulness.

Another methods are still under development to impart antibacterial function to the sheet masks for the sake of helping prevent and treat skin concerns, such as signs of aging, blemishes and even dermatitis, preserve the antibacterial function for a long period of time, and kill bacteria in the pores of the skin during facial massage or hydration.

KR Patent Laid-Open Publication No. 10-2017-0058687 discloses an antibacterial sheet mask using a serum containing gold particles. Korean Patent Application No. 10-2016-7010218 filed by Kuraray Co., Ltd. describes an antibacterial unwoven sheet made by texturing an antibacterial fiber and a solvent-spun cellulose fiber. In the antibacterial unwoven sheet, an antibacterial ethylene-vinyl alcohol copolymer containing inorganic antibacterial particles dispersed therein is present at least on part of the antibacterial fiber. The inorganic antibacterial particles have an average particle diameter of 0.01 to 20 µm, and the antibacterial ethylene-vinyl alcohol copolymer contains 10 to 70 mol.% of ethylene. Hence, the antibacterial unwoven sheet has excellent antiseptic and antibacterial functions without containing paraben or the like and displays good tactile qualities, such as feeling soft and pleasant to the touch. When impregnated with a variety of liquid compositions, the antibacterial unwoven sheet has excellent impregnation and liquid-retaining properties, allows the liquid compositions to be released by pressure with efficiency, but hardly undergoes deterioration of solidity or shrinkage caused by impregnation with the liquid compositions, thereby securing dimensional stability.

Yet, the antibacterial unwoven sheet uses expensive materials like gold and consists of an unwoven fabric, which ends up failing to form a tight seal against the face.

### DISCLOSURE OF INVENTION

### TECHNICAL PROBLEM

It is an object of the present invention to provide a novel conductive mask sheet.

It is another object of the present invention to provide a novel antibacterial mask sheet.

It is still another object of the present invention to provide a novel conductive and antibacterial mask sheet.

It is further another object of the present invention to provide a novel mask sheet that ensures a tight seal against the face and displays electrical conductivity and antibacterial function.

It is further another object of the present invention to provide a novel thin mask sheet that secures a tight seal against the face, displays electrical conductivity and antibacterial function and has good tactile quality.

It is further another object of the present invention to provide a method of manufacturing a novel thin mask sheet that secures a tight seal against the face, displays electrical conductivity and antibacterial function and has good tactile quality.

It is further another object of the present invention to provide a sheet mask using a mask sheet that has electrical conductivity and antibacterial function, forms a tight seal against the face and displays good tactile quality.

It is further another object of the present invention to provide a fabric material that has electrical conductivity and antibacterial function and ensures a snug fit.

It is further another object of the present invention to provide a method for manufacturing a woven fabric with electrical conductivity and antibacterial function.

### TECHNICAL SOLUTION

To achieve the objects of the present invention, there is provided a sheet mask that includes a knit mask sheet and a cosmetic composition soaking into the mask sheet, where the knit mask sheet includes 1 to 20 wt.% of an electrically conductive fiber containing a copper component.

Throughout this specification, the term "knit" is understood to imply that fabric is made with a circular knitting machine.

Throughout this specification, the term "knit fabric" refers to a fabric material resulting from knitting. The knit fabric can extend with a stretch percentage of at least 10 %, preferably at least 20 %, more preferably at least 30 %, and returns to its original length after stretching.

Throughout this specification, if an element is referred to as being "conductive" or "electrically conductive", it exhibits conductivity and allows electricity to travel through it.

Throughout this specification, if an element is referred to as being "antibacterial", it can inhibit the growth of at least one bacterium to at least 50% reduction, more preferably to at least 90% reduction.

Throughout this specification, if an element is referred to as being "thin", its thickness is 0.5 mm or less, more preferably 0.4 mm or less, and still more preferably 0.35 mm or less.

Throughout this specification, the term "low-denier fiber" refers to a fiber having a denier count between 15d and 150d.

Throughout this specification, the term "low-denier fabric" refers to a fabric containing a low-denier fiber in an amount of at least 60 wt.%, preferably at least 70 wt.%, more preferably at least 80 wt.%, still more preferably at least 90 wt.%, and most preferably at least 95 wt.%.

Throughout this specification, the term "eco-friendly mask sheet" refers to a mask sheet containing a natural fiber, for example, a natural cotton fiber in an amount of above 50 wt.%, and preferably at least 60 wt.%.

Throughout this specification, the term "eco-friendly mask sheet" refers to a mask sheet containing a natural fiber, for example, a natural cotton fiber in an amount of 20 to 50 wt.%.

In the present invention, the electrically conductive fiber containing a copper component is used to impart electrical conductivity and antibacterial function to fibers. Without theoretical limitations, when the mask sheet of the present invention is placed on the face, the copper component contained in the conductive fiber adheres to the face to make an antibacterial effect against various bacteria living on the skin of the face, strengthen antistatic properties thanks to the electrical conductivity and enhance hydrophilicity. Further, the mask sheet has good tactile quality due to a high content of the existing fibers and excellent elasticity resulting from knitting, thereby forming a tight seal against the face.

In the present invention, the electrically conductive fiber containing a copper component preferably consists of a fiber core and a copper component forming the surface thereof so that it can have a stretch together with the other fibers of the knit mask sheet and return to its original length after stretching. In the preferred embodiment of the present invention, the electrically conductive fiber containing a copper component may be a conductive fiber having a coating of a copper component on the surface of a nylon fiber in order to provide elasticity and rigidity required to the sheet mask. The conductive nylon fiber surface-coated with a copper component may be fabricated according to Korean Patent No. 10-1074692 granted to Jang et al., which patent is herein incorporated by reference in its entirety.

In the embodiment of the present invention, the conductive nylon fiber coated with a copper component may be a microfiber having a diameter of 1 to 1,000 µm, more preferably 50∼100 µm for making a thin sheet mask.

In the embodiment of the present invention, the electrically conductive fiber surface-coated with a copper component may be used in an amount of at least 1 wt.%, preferably at least 3 wt.%, and more preferably at least 5 wt.%, in order to impart electrical conductivity and antibacterial function to the sheet mask. Further, if used in excess, the electrically conductive fiber surface-coated with a copper component results in deterioration of the snug fit of the mask due to the odor from the copper component and does not increase the electrical conductivity in proportion to its amount; thus, it is preferably used in an amount of 20 wt.% or less, preferably 15 wt.% or less. In a preferred embodiment of the present invention, the electrically conductive fiber surface-coated with a copper component is used in an amount of 5 to 15 wt.%.

In the present invention, the other fiber as used herein may be at least one non-conductive fiber. The non-conductive fiber may be a natural fiber, a synthetic fiber, or a combination of both. In the embodiment of the present invention, the natural fiber may be cotton fiber and/or Tencel fiber; and the synthetic fiber may be polyester fiber, polyurethane fiber such as spandex, or antibacterial fiber containing an antibacterial agent.

In the present invention, the mask sheet may be an textured knitting sheet consisting of a conductive region knitted using an electrically conductive fiber containing copper and a non-conductive region knitted using other fibers. The conductive region is to impart electrical conductivity required to the mask sheet by using the copper-containing conductive fiber in a small amount of 1 to 20 wt.% with respect to the total weight of the fabric.

In the present invention, the conductive region may be a defined conductive pattern formed in the non-conductive region, preferably a conductive pattern extending from the one side to the other of the fabric. The pattern may be a striped pattern with stripes spaced at a defined distance from each other, allowing electricity to travel along the continuous conductive stripes.

In the present invention, the mask sheet is preferably a mask sheet made of a fabric treated by a scouring process for eliminating impurities from the conductive fiber containing copper and the non-conductive fiber to enhance the tactile quality. In the embodiment of the present invention, the scouring process is carried out preferably at a near-neutral weak alkaline value of pH 8.0 or less, more preferably at pH 7.5 to 8.0, in order to prevent electrical conductivity and antibacterial function from deteriorating with a desorption of copper.

In the present invention, the mask sheet may come in different colors through dyeing. According to the embodiment of the present invention, the mask sheet may be dyed in white or black. In the embodiment of the present invention, it is preferable to mix the other fibers with a dye and spin them for fear that the dye component become eluted with the cosmetic component soaking into the mask sheet.

In the present invention, the mask sheet may be an eco-friendly mask sheet that includes an electrically conductive fiber containing a copper component.

In the present invention, the eco-friendly mask sheet consists of an electrically conductive fiber containing a copper component and a natural fiber. In the embodiment of the present invention, the eco-friendly mask sheet may include 1 to 20 wt.% of the electrically conductive fiber containing a copper component and 80 to 99 wt.% of a cotton fiber.

The eco-friendly mask sheet may be a mask sheet using a natural cotton fiber derived from cotton. The cotton fiber provides eco-friendliness of a natural fiber and soft feel of cotton for the sheet mask. The diameter of the cotton fiber may be appropriately adjusted under necessity. For proper rigidity and soft touch, the natural cotton fiber preferably has a cotton count ranging from 30s to 100s, for example, 30s, 40s, 50s, 60s, 70s, 80s, or 100s. In the preferred embodiment of the present invention, the cotton fiber may be a cotton fiber with proper fineness and a cotton count ranging from 40s to 60s, where 40s corresponds to 100 denier, and 60s corresponds to 88 denier.

In one aspect of the present invention, the mask sheet may be an eco-friendly antibacterial mask sheet. In the present invention, the eco-friendly antibacterial mask sheet consists of an electrically conductive fiber containing a copper component, a non-conductive fiber containing a copper component for strengthening the antibacterial function, and a natural fiber. In the embodiment of the present invention, the eco-friendly antibacterial mask sheet may include 1 to 20 wt.% of the electrically conductive fiber containing a copper component, 1 to 20 wt.% of the non-conductive fiber containing a copper component, and 60 to 98 wt.% of a cotton fiber.

The cotton fiber used in the eco-friendly antibacterial mask sheet is substantially the same as that used in the eco-friendly mask sheet. Apart from the conductive fiber containing a copper component, the non-conductive fiber containing a copper component is used to impart additional antibacterial function to the sheet mask.

The copper component contained in the non-conductive fiber adheres to the surface of the fiber in an intermittent manner to impart antibacterial function to the fiber. Yet, the fiber is not conductive. The non-conductive fiber containing a copper component is a commercially available fiber; for example, Glotech fiber (Kolon Industries Inc., South Korea). In the embodiment of the present invention, the non-conductive fiber containing a copper component may be used in an amount of at least 1 wt.%, preferably at least 3 wt.%, and more preferably at least 5 wt.%.

Further, the electrically conductive fiber surface-coated with a copper component may be used in an amount of 20 wt.% or less, preferably 15 wt.% or less, for the sake of preventing an odor of copper generated from the mask sheet. In the preferred embodiment of the present invention, the electrically conductive fiber surface-coated with a copper component is used in an amount of 5 to 15 wt.%. Preferably, the electrically non-conductive fiber containing copper has a fineness (denier count) ranging from 50d to 100d as suitable for use in a thin mask sheet.

In one aspect of the present invention, the mask sheet may be a thin antibacterial mask sheet. In the present invention, the thin antibacterial mask sheet may consist of low-denier fibers to ensure a tight seal to the face and a soft feel, preferably polyester and spandex fibers, so that it maintain high elasticity despite being as thin as a film but make no wrinkle due to the difference of shrinkage percentage between the textured knitting regions during the process of impregnation with a cosmetic composition. In the embodiment of the present invention, the thin antibacterial mask sheet may include 1 to 20 wt.% of the electrically conductive fiber containing a copper component, 1 to 20 wt.% of the non-conductive fiber containing a copper component, 50 to 90 wt.% of the polyester fiber, and 1 to 20 wt.% of the spandex fiber.

The cotton fiber and the non-conductive fiber containing a copper component are substantially the same as those used in the eco-friendly antibacterial mask sheet. The polyester fiber is used to provide dimensional stability for the mask sheet so that the mask sheet retain its original shape without changing in shape by the liquid cosmetic composition soaking into it. The polyester fiber may be used in an amount of preferably 50 to 90 wt.%, more preferably 60 to 85 wt.%, and still more preferably 70 to 80 wt.%. The content of the polyester fiber below the defined range results in wrinkles on the surface of the mask sheet after soaking the mask sheet with the cosmetic composition; and the content of the polyester fiber above the defined range reduces the relative content of the other fibers to deteriorate electrical conductivity and antibacterial function.

The spandex fiber is commercially available and used not only to provide dyeability and dimensional stability for the sheet mask but also to ensure a snug fit so that the sheet mask fit more tightly to the face. The spandex fiber is preferably contained in an amount of 1 to 20 wt.%, and more preferably 5 to 15 wt.%.

In one aspect of the present invention, the mask sheet may be a thin eco-friendly antibacterial mask sheet. The thin eco-friendly antibacterial mask sheet of the present invention may use cotton fibers as a natural material for eco-friendliness, and further an electrically conductive fiber containing a copper component, a non-conductive fiber containing a copper component, a polyester fiber, a spandex fiber, and a Tencel fiber, which fibers are used to compensate for the relatively low rigidity of the cotton fiber in relation to the synthetic fibers' rigidity, to help the thin sheet mask maintain high elasticity despite being as thin as a film and to keep it from wrinkling due to the difference of shrinkage percentage between the textured knitting regions during the process of soaking with a cosmetic composition.

In the embodiment of the present invention, the thin eco-friendly antibacterial mask sheet may include 1 to 20 wt.% of the electrically conductive fiber containing a copper component, 1 to 20 wt.% of the non-conductive fiber containing a copper component, 10 to 40 wt.% of the cotton fiber, 10 to 30 wt.% of the Tencel fiber, 20 to 50 wt.% of the polyester fiber, and 1 to 20 wt.% of the spandex fiber.

The electrically conductive fiber containing a copper component and the non-conductive fiber containing a copper component are substantially the same as those used in the thin antibacterial mask sheet. The cotton fiber may be contained in an amount of 10 to 40 wt.%. The content of the cotton fiber below the defined range results in a failure to impart the characteristic features of the cotton fiber to the mask sheet; and the content of the cotton fiber above the defined range deteriorates the dimensional stability of the mask sheet due to the cotton fiber having a relatively lower rigidity than the other fibers when soaked in moisture, ending up with the mask sheet being creased. In the embodiment of the present invention, if the cotton fiber is a low-denier cotton fiber, its content is more preferably 15 to 35 wt.%, and most preferably 20 to 30 wt.%.

The Tencel fiber, which is a regenerated cellulose fiber formed from wood pulp, is used in the mask sheet to raise the content of natural components and compensate for the relatively low wetting strength of the cotton fiber. The Tencel fiber is contained in an amount of 10 to 30 wt.%. The content of the Tencel fiber below the defined range results in a failure to impart the characteristic features of the mask sheets having a high content of natural materials; and the content of the Tencel fiber above the defined range reduces the relative content of the synthetic fibers and eventually deteriorates the dimensional stability of the mask. In the embodiment of the present invention, if the Tencel fiber is a low-denier Tencel fiber, its content is more preferably 12 to 28 wt.%, and most preferably 15 to 25 wt.%.

The polyester fiber is used to provide dimensional stability so that the mask sheet has no change in shape by the liquid cosmetic composition used for soaking it. The polyester fiber is preferably contained in an amount of 20 to 50 wt.%, more preferably 25 to 45 wt.%, and still more preferably 30 to 40 wt.%. The content of the polyester fiber reduced below the defined range deteriorates the entire dimensional stability and results in forming wrinkles on the surface of the mask sheet after soaking with the cosmetic composition; and the content of the polyester fiber increased to excess the defined range reduces the relative content of the other fibers to lower the content of the natural components.

The spandex fiber is commercially available and used not only to provide dyeability and dimensional stability for the sheet mask but also to ensure a snug fit resulting from the characteristics of spandex so that the sheet mask fit more tightly to the face. The spandex fiber is preferably contained in an amount of 1 to 20 wt.%, and more preferably 5 to 15 wt.%.

In the present invention, the mask sheet is a cut of the knit fabric sheet having a high elasticity so that the users can stretch the mask sheet in accord with their face contour. If the sheet is woven, it is not easy to stretch because the warp and weft are tightening up each other. In the embodiment of the present invention, the knit fabric can extend with a stretch percentage of at least 50 % and return to its original length after stretching.

In the present invention, when the fabrics constituting the mask sheet are for use in a thin mask sheet, they may use fibers having a denier count ranging from 15d to 150d. The denier count of the fibers for thin mask sheet may be adjusted to be suitable for the texture depending on the fiber type; preferably, 50d to 100d for an electrically conductive fiber containing copper, 50d to 150d for cotton, 50d to 150d for Tencel, 50d to 75d for polyester, and 15d to 50d for spandex.

In one aspect of the present invention, there is provided a sheet mask that includes a mask sheet and a cosmetic composition, the mask sheet being a knit sheet cut into the shape of a face and comprised of an electrically conductive fiber containing a copper component and other fibers. The mask sheet includes 1 to 20 wt.% of the electrically conductive fiber containing a copper component, 1 to 20 wt.% of a non-conductive fiber containing a copper component, and 60 to 98 wt.% of a cotton fiber.

In another aspect of the present invention, there is provided a sheet mask that includes a mask sheet and a cosmetic composition, the mask sheet being a knit sheet cut into the shape of a face and comprised of an electrically conductive fiber containing a copper component, a non-conductive fiber containing a copper component, a polyester fiber, and a spandex fiber. The fibers are low-denier fibers having a denier count ranging from 15d to 150d. The mask sheet includes 1 to 20 wt.% of the electrically conductive fiber containing a copper component, 1 to 20 wt.% of the non-conductive fiber containing a copper component, 50 to 90 wt.% of the polyester fiber, and 1 to 20 wt.% of the spandex fiber.

In still another aspect of the present invention, there is provided a sheet mask that includes a mask sheet and a cosmetic composition, the mask sheet being a knit sheet cut into the shape of a face and comprised of a fiber containing a copper component, a cotton fiber, a Tencel fiber, a polyester fiber, and a spandex fiber. The fibers are low-denier fibers having a denier count ranging from 15d to 150d. The fiber containing a copper component is an electrically conductive fiber, a non-conductive fiber, or a combination of both. The mask sheet includes 2 to 40 wt.% of the fiber containing a copper component, 10 to 40 wt.% of the cotton fiber, 10 to 30 wt.% of the Tencel fiber, 20 to 50 wt.% of the polyester fiber, and 1 to 20 wt.% of the spandex fiber.

In still another aspect of the present invention, there is provided a sheet mask that includes a knit mask sheet comprised of 1 to 20 wt.% of an electrically conductive fiber containing a copper component and a cosmetic composition soaking into the mask sheet. The cosmetic composition contains a copper component. Without theoretical limitations, the copper component contained in the electrically conductive fiber is eluted in the form of ions into the liquid cosmetic composition such as serum. The copper component may be contained in an amount of at least 1 ppm, preferably at least 10 ppm, and more preferably 20 to 100 ppm per gram of the cosmetic composition.

In one aspect of the present invention, there is provided a method for manufacturing a conductive and antibacterial sheet mask that includes: knitting a fabric using a conductive fiber containing a copper component and a cotton fiber; cutting the knit fabric into the shape of a face to make a mask sheet; and soaking the mask sheet in a serum.

In the present invention, the method for manufacturing a conductive and antibacterial sheet mask further includes scouring the knit fabric. In the embodiment of the present invention, the scouring process may be carried out preferably at a near-neutral weak alkaline value of pH 8.0 or less, more preferably at pH 7.5 to 8.0, in order to prevent electrical conductivity and antibacterial function from deteriorating due to a desorption of copper during the process.

In another aspect of the present invention, there is provided a skincare method that includes placing a knit mask sheet including 1 to 20 wt.% of an electrically conductive fiber containing a copper component on the face; and applying electric current to the mask sheet.

In still another aspect of the present invention, there is provided a therapeutic method that includes placing a knit mask sheet including 1 to 20 wt.% of an electrically conductive fiber containing a copper component on the face; and applying electric current to the mask sheet.

In still further another aspect of the present invention, there is provided a knit fabric with electrical conductivity and antibacterial function that includes 1 to 20 wt.% of an electrically conductive fiber containing a copper component.

### EFFECTS OF INVETNION

The present invention provides a novel functional mask sheet with antibacterial function and/or electrical conductivity and a sheet mask using the functional mask sheet.

The sheet mask of the present invention can avoid or minimize the use of a preservative because of its antibacterial function. The antibacterial function of the mask sheet also relieves skin irritations caused by bacterial infection.

Further, the electrical conductivity of the mask sheet imparts antistatic function to the sheet and further enhances the absorbency of the sheet for the beneficial ingredients through electric stimulation.

Further, the thin functional mask sheet of the present invention feels soft and highly elastic and hence secures a snug fit.

Further, the thin functional mask sheet of the present invention is beneficial to skincare due to the liquid cosmetic composition containing a copper component.

Furthermore, the thin functional fabric of the present invention maintains its antibacterial function and electrical conductivity even after washed dozens of times.

### BRIEF DESCRIPTION OF DRAWINGS;

FIG. 1 is a photographic image of a mask sheet according to one embodiment of the present invention.
FIG. 2 presents photographic images showing the electrical conductivity of a knit fabric according to one embodiment of the present invention.
FIG. 3 is a test report showing the antibacterial function of a knit fabric according to one embodiment of the present invention.
FIG. 4 shows the elasticity of a fabric for mask sheet according to one embodiment of the present invention; FIG. 4a is the fabric before stretching and FIG. 4b is the fabric stretched on both sides.
FIG. 5 is a photographic image of a mask sheet according to another embodiment of the present invention.
FIG. 6 is a photographic image of a mask sheet according to still another embodiment of the present invention.
FIG. 7 shows the thickness measurement of a mask sheet according to still another embodiment of the present invention.
FIG. 8 is a test report showing the antibacterial function of a knit fabric according to one embodiment of the present invention after laundry.

### BEST MODES FOR CARRYING OUT THE INVENTION

Hereinafter, the present invention will be described in further detail with reference to examples. It will be obvious to those skilled in the art that these examples are illustrative purposes only and are not intended to limit the scope of the present invention.

### Example 1

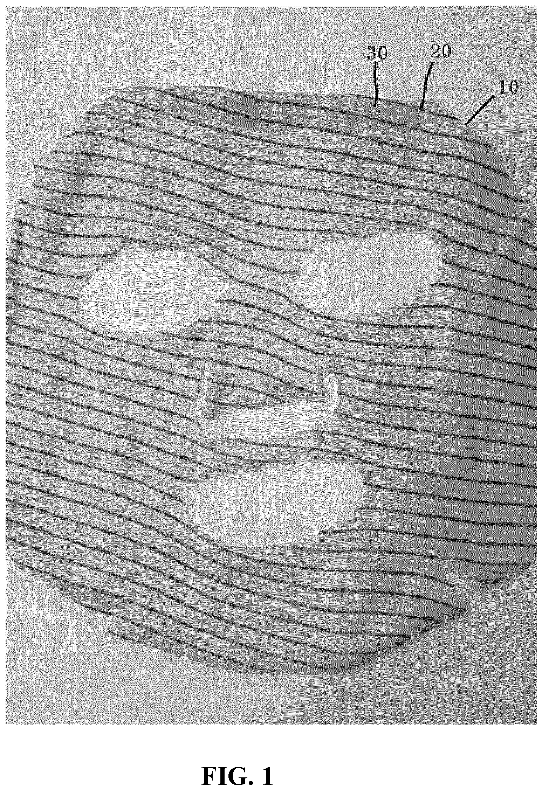

A knitting machine was used to make a knit fabric consisting of 5 wt.% of a copper-containing conductive 60-denier nylon fiber, 5 wt.% of a 70-denier Glotech fiber, and 90 wt.% of a 130-denier cotton fiber in a striped pattern with alternating stripes of the copper-containing conductive nylon fiber and the Glotech fiber. The knit fabric was scoured and cut into the shape of a face as shown in FIG. 1 to prepare a mask sheet. Referring to FIG. 1, on the cotton base 10 were formed alternating stripes of the copper-containing conductive nylon fiber 20 and the non-conductive nylon fiber 30.

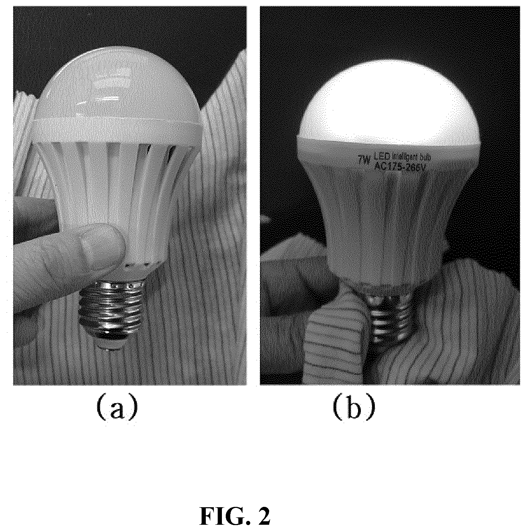

The socket of a light bulb for measuring electrical conductivity was wrapped in the scoured knit mask sheet and plugged on. As can be seen from FIG. 2, the bulb lighted up.

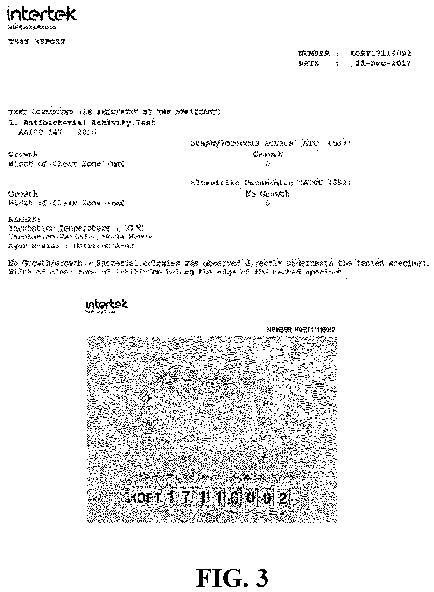

The antibacterial function of the scoured knit fabric was measured. As shown in FIG. 3, the scoured knit fabric proved to have antibacterial function to 100% reduction against *Staphylococcus Aureus* and *Klebsiella Pneumoniae.*

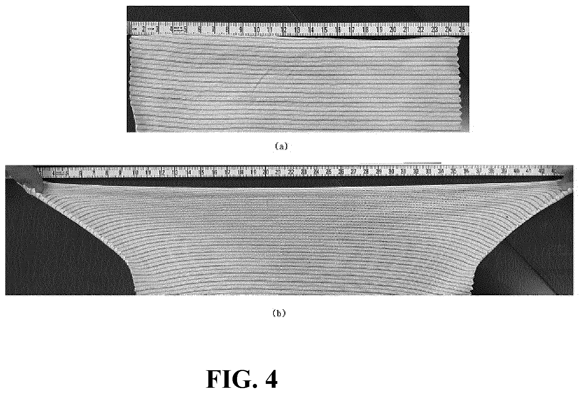

The scoured knit fabric was also measured in regards to the elasticity. As shown in FIG. 4, the knit fabric was stretched.

The mask sheet thus obtained was soaked in a serum and placed in a packaging container to complete a sheet mask.

### Example 2

A knitting machine was used to make a knit fabric including 10 wt.% of the copper-containing conductive nylon fiber, 10 wt.% of the Glotech fiber, and 80 wt.% of the 130-denier cotton fiber in a striped pattern with alternating stripes of the copper-containing conductive nylon fiber and the Glotech fiber. The knit fabric was scoured and cut into the shape of a face to complete a mask sheet.

### Comparative Example 1

The procedures were performed in the same manner as described in Example 1, excepting that the knit fabric used 30 wt.% of the copper-containing conductive nylon fiber, 10 wt.% of the Glotech fiber, and 60 wt.% of the 130-denier cotton fiber.

### Comparative Example 2

The procedures were performed in the same manner as described in Example 1, excepting that the knit fabric was not scoured.

**[Table 1]**

| Div. | Examples | | Comparative Examples | |
|---|---|---|---|---|
| | 1 | 2 | 1 | 2 |
| Thinness | ○ | ○ | ○ | ○ |
| Touch quality | ○ | ○ | ○ | X |
| Smell | ○ | ○ | X | ○ |
| Impregnation state | (○) | (○) | (○) | (○) |
| Electrical conductivity | (○) | (○) | (○) | (○) |

| | | | | |
|---|---|---|---|---|
| (○) Excellent: 8 out of 10 panels called good, ○ Fair: 6 out of 10 panels called good, Δ Bad: 4 out of 10 panels called good, X Worst: 2 out of 10 panels called good. Thinness: Panels determined whether the sheet mask placed on the face felt thin and clear. Touch quality: Panels determined whether the mask sheet felt soft to the touch. Smell: Panels determined whether the mask sheet smelled a coppery odor. Impregnation state: From visual observation one hour after soaking a sample of the mask sheet specimen in serum, panels called good if no uneven surface was observed; and called bad if uneven surface was observed. Electrical conductivity: Panels determined whether the bulb wrapped in the mask sheet lighted up. | | | | |

### Example 3

The copper-containing conductive nylon fiber, the Glotech fiber, a 50-denier black polyester fiber, and a 30-denier black spandex fiber were arranged in a knitting machine. With the loop length of each fiber adjusted, the knitting machine was operated to form alternating stripes of the conductive nylon fiber and the Glotech fiber by knitting alternately with the polyester and spandex fibers.

5 wt.% of the copper-containing conductive nylon fiber, 5 wt.% of the Glotech fiber, 85 wt.% of the polyester fiber, and 5 wt.% of the spandex fiber were used to make a fabric in a striped pattern by textured knitting. The fabric was then scoured to complete a striped knit fabric.

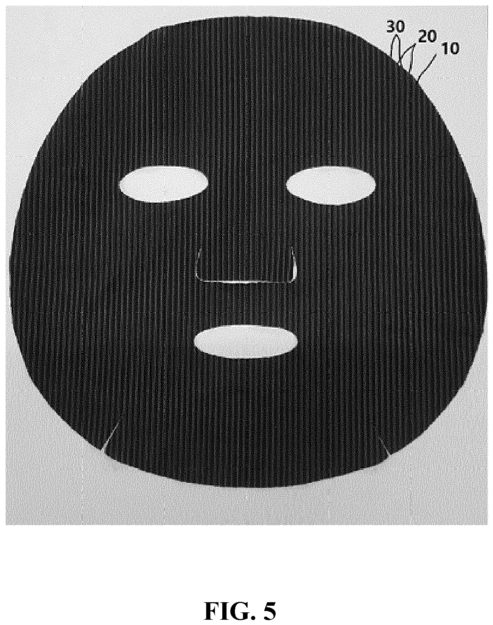

The knit fabric thus obtained was cut into the shape of a face to make a mask sheet as shown in FIG. 5.

Referring to FIG. 5, on the polyester-spandex blend base 10 were formed alternating stripes of the copper-containing conductive nylon fiber 20 and the copper-containing non-conductive nylon fiber 30. The mask sheet thus obtained was soaked in a serum and put into a packaging container to complete a sheet mask.

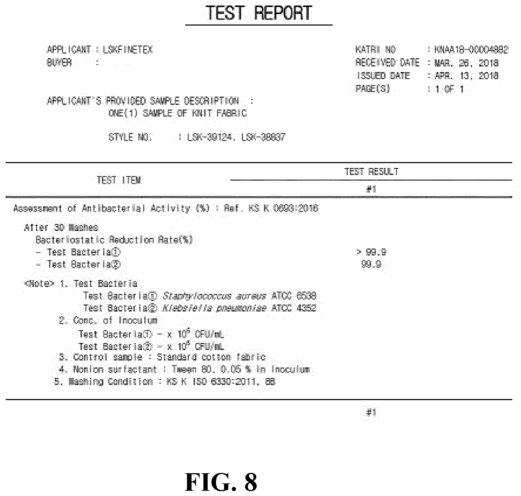

The sheet mask was left untouched for 30 days and measured in regards to the concentration of copper in the serum. The detected amount of the copper component was 42 µg/g per unit of serum. The antibacterial function of the knit fabric was evaluated after laundry. As shown in FIG. 8, the knit fabric proved to have antibacterial function to 99% reduction against *Staphylococcus Aureus* and *Klebsiella Pneumoniae* even after 30 times of laundry.

### Example 4

The procedures were performed in the same manner as described in Example 3, excepting that the knit fabric used 7 wt.% of the copper-containing conductive nylon fiber, 7 wt.% of the Glotech fiber, 76 wt.% of the polyester fiber, and 10 wt.% of the spandex fiber.

### Example 5

The procedures were performed in the same manner as described in Example 3, excepting that the knit fabric used 15 wt.% of the copper-containing conductive nylon fiber, 15 wt.% of the Glotech fiber, 55 wt.% of the polyester fiber, and 15 wt.% of the spandex fiber.

### Example 6

The procedures were performed in the same manner as described in Example 3, excepting that a 15-denier polyester fiber was used.

### Example 7

The procedures were performed in the same manner as described in Example 3, excepting that a 150-denier polyester fiber was used.

### Comparative Example 3

The procedures were performed in the same manner as described in Example 3, excepting that the knit fabric used 25 wt.% of the copper-containing conductive nylon fiber, 25 wt.% of the Glotech fiber, 45 wt.% of the polyester fiber, and 5 wt.% of the spandex fiber.

### Comparative Example 4

The procedures were performed in the same manner as described in Example 3, excepting that the knit fabric used 0.5 wt.% of the copper-containing conductive nylon fiber, 0.5 wt.% of the Glotech fiber, 95 wt.% of the polyester fiber, and 4 wt.% of the spandex fiber.

### Comparative Example 5

The procedures were performed in the same manner as described in Example 3, excepting that 5-denier polyester and spandex fibers were used.

### Comparative Example 6

The procedures were performed in the same manner as described in Example 3, excepting that 200-denier polyester and spandex fibers were used.

### Comparative Example 7

The procedures were performed in the same manner as described in Example 3, excepting that the knit fabric was not scoured.

**[Table 2]**

| Div. | Examples | | | | | Comparative Examples | | | | |
|---|---|---|---|---|---|---|---|---|---|---|
| | 3 | 4 | 5 | 6 | 7 | 3 | 4 | 5 | 6 | 7 |
| Thinness | (○) | (○) | (○) | (○) | ○ | (○) | (○) | (○) | X | (○) |
| Touch quality | (○) | (○) | (○) | (○) | ○ | ○ | (○) | (○) | X | X |
| Smell | ○ | ○ | ○ | ○ | ○ | X | ○ | ○ | ○ | ○ |
| Impregnation state | (○) | (○) | (○) | (○) | (○) | Δ | (○) | X | (○) | (○) |
| Electrical conductivity | (○) | (○) | (○) | (○) | (○) | (○) | X | (○) | (○) | (○) |

| | | | | | | | | | | |
|---|---|---|---|---|---|---|---|---|---|---|
| (○) Excellent: 8 out of 10 panels called good, ○ Fair: 6 out of 10 panels called good, Δ Bad: 4 out of 10 panels called good, X Worst: 2 out of 10 panels called good. Thinness: Panels determined whether the sheet mask placed on the face felt thin and clear. Touch quality: Panels determined whether the mask sheet felt soft to the touch. Smell: Panels determined whether the mask sheet smelled a coppery odor. Impregnation state: From visual observation one hour after soaking a sample of the mask sheet specimen in serum, panels called good if no uneven surface was observed; and called bad if uneven surface was observed. Electrical conductivity: Panels determined whether the bulb wrapped in the mask sheet lighted up. | | | | | | | | | | |

### Example 8

The conductive nylon fiber, the Glotech fiber, a cotton fiber, a Tencel fiber, a polyester fiber, and a black spandex fiber were arranged in a knitting machine. With the loop length of each fiber adjusted, the knitting machine was operated to form alternating strips of the conductive nylon fiber and the Glotech fiber by knitting alternately with a yarn consisting of 100-denier cotton fiber, 100-denier Tencel fiber, 50-denier polyester fiber, and 30-denier spandex fiber.

5 wt.% of the copper-containing conductive nylon fiber, 5 wt.% of the Glotech fiber, 25 wt.% of the cotton fiber, 25 wt.% of the Tencel fiber, 30 wt.% of the polyester fiber, and 10 wt.% of the spandex fiber were used to make a fabric in a striped pattern by textured knitting. The fabric was then scoured to complete a striped knit fabric.

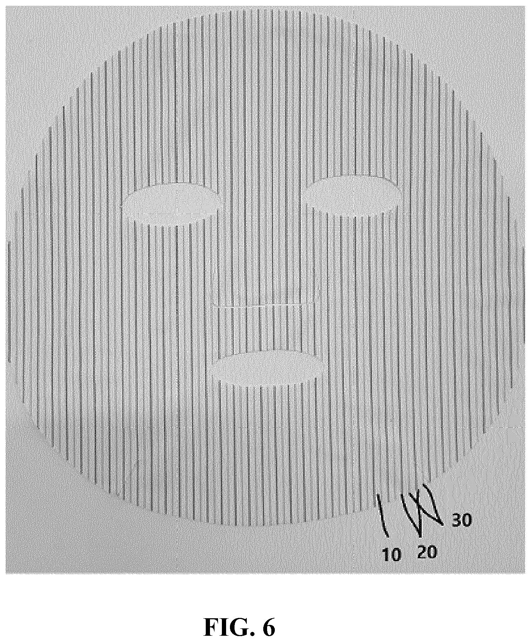

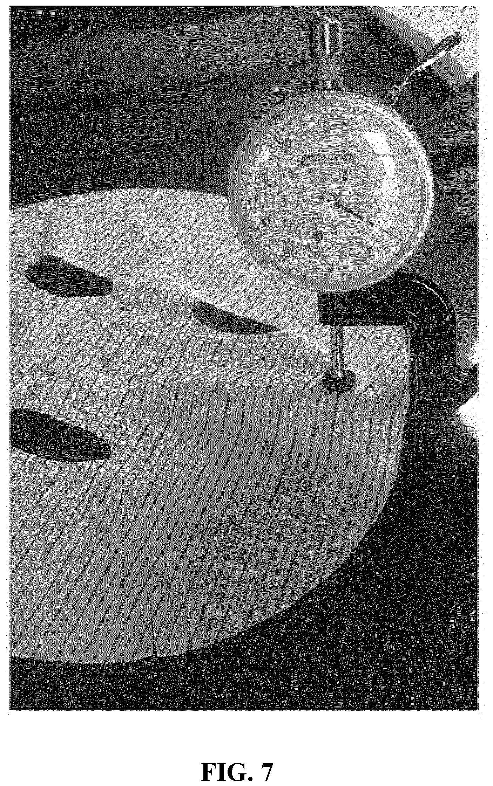

The knit fabric thus obtained was cut into the shape of a face to make a mask sheet as shown in FIG. 6. Referring to FIG. 6, on the polyester-spandex-cotton- Tencel blend base 10 were formed alternating stripes of the copper-containing conductive nylon fiber 20 and the copper-containing non-conductive nylon fiber 30. As shown in FIG. 7, the knit fabric was measured in regards to the thickness, and the thickness measurement was 0.35 mm. The mask sheet thus obtained was soaked in a serum and put into a packaging container to complete a sheet mask.

The sheet mask was left untouched for 30 days and measured in regards to the concentration of copper in the serum. The detected amount of the copper component was 30 µg/g per unit of serum. The antibacterial function of the knit fabric was evaluated after laundry.

### Example 9

The procedures were performed in the same manner as described in Example 8, excepting that the knit fabric used 20 wt.% of the conductive nylon fiber, 5 wt.% of the Glotech fiber, 20 wt.% of the cotton fiber, 20 wt.% of the Tencel fiber, 30 wt.% of the polyester fiber, and 5 wt.% of the spandex fiber.

### Example 10

The procedures were performed in the same manner as described in Example 8, excepting that the knit fabric used 5 wt.% of the conductive nylon fiber, 5 wt.% of the Glotech fiber, 30 wt.% of the cotton fiber, 15 wt.% of the Tencel fiber, 40 wt.% of the polyester fiber, and 5 wt.% of the spandex fiber.

### Example 11

The procedures were performed in the same manner as described in Example 8, excepting that a 150-denier polyester fiber was used.

### Comparative Example 8

The procedures were performed in the same manner as described in Example 8, excepting that the knit fabric used 5 wt.% of the copper-containing conductive nylon fiber, 5 wt.% of the Glotech fiber, 50 wt.% of the cotton fiber, 20 wt.% of the Tencel fiber, 15 wt.% of the polyester fiber, and 5 wt.% of the spandex fiber.

### Comparative Example 9

The procedures were performed in the same manner as described in Example 8, excepting that the knit fabric used 5 wt.% of the copper-containing conductive nylon fiber, 5 wt.% of the Glotech fiber, 30 wt.% of the cotton fiber, 20 wt.% of the Tencel fiber, 25 wt.% of the polyester fiber, and 15 wt.% of the spandex fiber.

### Comparative Example 10

The procedures were performed in the same manner as described in Example 8, excepting that the knit fabric used 5 wt.% of the copper-containing conductive nylon fiber, 5 wt.% of the Glotech fiber, 50 wt.% of the cotton fiber, 30 wt.% of the polyester fiber, and 10 wt.% of the spandex fiber.

### Comparative Example 11

The procedures were performed in the same manner as described in Example 8, excepting that the knit fabric used 5 wt.% of the copper-containing conductive nylon fiber, 5 wt.% of the Glotech fiber, 50 wt.% of the cotton fiber, 30 wt.% of the polyester fiber, and 10 wt.% of the spandex fiber.

### Comparative Example 12

The procedures were performed in the same manner as described in Example 8, excepting that the knit fabric used 5 wt.% of the copper-containing conductive nylon fiber, 5 wt.% of the Glotech fiber, 50 wt.% of the cotton fiber, 25 wt.% of the Tencel fiber, and 15 wt.% of the spandex fiber.

### Comparative Example 13

The procedures were performed in the same manner as described in Example 8, excepting that the knit fabric used 25 wt.% of the copper-containing conductive nylon fiber, 20 wt.% of the Glotech fiber, 20 wt.% of the cotton fiber, 10 wt.% of the Tencel fiber, 20 wt.% of the polyester fiber, and 5 wt.% of the spandex fiber.

### Comparative Example 14

The procedures were performed in the same manner as described in Example 8, excepting that a 200-denier polyester fiber was used.

### Comparative Example 15

The procedures were performed in the same manner as described in Example 8, excepting that the knit fabric was not scoured.

**[Table 3]**

| Div. | Examples | | | | Comparative Examples | | | | | | | |
|---|---|---|---|---|---|---|---|---|---|---|---|---|
| | 8 | 9 | 10 | 11 | 8 | 9 | 10 | 11 | 12 | 13 | 14 | 15 |
| Thinness | (○) | (○) | (○) | (○) | ○ | (○) | (○) | (○) | ○ | (○) | X | (○) |
| Touch quality | (○) | (○) | (○) | (○) | ○ | ○ | (○) | (○) | (○) | (○) | X | X |
| Smell | ○ | ○ | ○ | ○ | ○ | X | ○ | ○ | ○ | X | ○ | X |
| Impregnatio n state | (○) | (○) | (○) | (○) | X | Δ | X | X | X | (○) | (○) | (○) |

| | | | | | | | | | | | | |
|---|---|---|---|---|---|---|---|---|---|---|---|---|
| (○) Excellent: 8 out of 10 panels called good, ○ Fair: 6 out of 10 panels called good, Δ Bad: 4 out of 10 panels called good, X Worst: 2 out of 10 panels called good. Thinness: Panels determined whether the sheet mask placed on the face felt thin and clear. Touch quality: Panels determined whether the mask sheet felt soft to the touch. Smell: Panels determined whether the mask sheet smelled a coppery odor. Impregnation state: From visual observation one hour after soaking a sample of the mask sheet in serum, panels called good if no uneven surface was observed; and called bad if uneven surface was observed. Electrical conductivity: Panels determined whether the bulb wrapped in the mask sheet lighted up. | | | | | | | | | | | | |

### [Experiments]

### Copper-containing conductive nylon fiber

According to the embodiment of KR Patent No. 10-1167860, an about 70-denier nylon fiber was put into a reaction bath. With a bath ratio adjusted to 1:15∼20, 3 to 5 g/L of a deoiling agent was added to the reaction bath at 60 °C for 40 min, and the suspended solids produced by the action of the deoiling agent were washed off with water. Into the reaction bath were added 2 to 3 % o.w.f. of levulinic acid, 0.1 to 0.5 % o.w.f. of sodium lauryl sulfate, 0.1 to 0.3 % o.w.f. of polyethylene glycol, 8 to 10 % o.w.f. of sodium thiosulfate, 0.2 % o.w.f. of EDTA, and 10 to 20 % o.w.f. of copper sulfate. After 10-minute agitation, the bath was heated up to 60 °C at a rate of 1 °C/min and a pH value ranging from 4 to 5. The reaction was activated at 60 °C for 60 min, and 0.3 % o.w.f. of sodium hypophosphite was added. Reductive precipitation was caused at 60 °C for 20 min, and unreacted substances were removed while the pH value was below 7. At pH 7, 0.3 % o.w.f. of magnesium hydroxide was added and activated at 40 °C for 20 min to remove the remaining sulfur. The conductive nylon fiber thus obtained had a specific resistance of 10² Ω·cm and a fineness of 60 denier.

### Non-conductive fiber

The copper-containing non-conductive fiber was 70-denier Glotech fiber (Kolon Industries Inc., South Korea). Cotton fiber, Tencel fiber, polyester fiber, and spandex fiber were commercially available.

### Electrical conductivity Test

For an electrical conductivity test, a light bulb was put into contact with the mask sheet. A connection of the bottom and lateral sides of the bulb socket to a conductive material causes the bulb to light up. As shown in FIG. 4, the bottom and lateral sides of the bulb socket were connected to the fabric. When the bulb was plugged on and lighted up, then the fabric proved to be electrically conductive.

### Antibacterial function test

An institute for antimicrobial efficacy testing, Intertek, was asked to evaluate the antibacterial function of the knit fabrics according to the AATCC 147:2016 method. The antibacterial function was measured against *Staphylococcus Aureus* (ATTC 6538) and *Klebsiella Pneumoniae* (ATTC 4352) after incubation of 18 to 24 hours in a nutrient medium at 37 °C.

### Antibacterial function after laundry

The antibacterial function test was conducted for the fabrics according to KS K 0693:2016. The antibacterial function was measured against *Staphylococcus Aureus* (ATTC 6538) and *Klebsiella Pneumoniae* (ATTC 4352). The concentration of the inoculated solution was 1.2x10⁵ CFU/mL for *Staphylococcus Aureus* (ATTC 6538) and 1.0x10⁵ CFU/mL for *Klebsiella Pneumoniae* (ATTC 4352). The control surface was the standard cotton fabric. Tween 80 was used as a nonionic surfactant for laundry, and its used amount was 0.05 % of the inoculated solution. Laundry conditions were given as specified in KS K ISO 6330: 2011, 8B.

### Preparation of sheet mask

The fabric was cut into the shape of a mask to obtain a mask sheet. Then, the mask sheet was soaked in a serum and placed in a packaging container to complete a sheet mask.

### Stretchability of mask sheet

The conductive fabric was pulled on both sides to stretch in the lengthwise direction of the stripes and measured in regards to the stretchability.

### Testing for thinness, touch quality and smell of mask sheet

The thickness of the mask sheet was measured with a fabric thickness gauge. The mask sheet, placed on the face, was subjected to a sensory evaluation in regards to thinness, touch quality, and smell. 10 panels were selected to participate in the testing for thinness, tough quality and smell of the mask sheet.

### Copper concentration

KOTITI was asked to measure the concentration of copper in the mask sheet according to In House Method (ICP-MS).

## Claims

1. A sheet mask comprising a knit mask sheet and a cosmetic composition soaking into the mask sheet,
the knit mask sheet comprising 1 to 20 wt.% of an electrically conductive fiber containing a copper component.

2. The sheet mask as claimed in claim 1, wherein the mask sheet comprises a conductive fiber containing a copper component and a non-conductive fiber.

3. The sheet mask as claimed in claim 1 or 2, wherein the mask sheet comprises a conductive region knitted using the electrically conductive fiber containing copper and a non-conductive region knitted using other fibers.

4. The sheet mask as claimed in claim 3, wherein the conductive region has a striped pattern.

5. The sheet mask as claimed in claim 1 or 2, wherein the mask sheet is a scoured mask sheet.

6. The sheet mask as claimed in claim 1 or 2, wherein the mask sheet comprises a low-denier fiber having a denier count between 15d and 150d.

7. The sheet mask as claimed in claim 1 or 2, wherein the mask sheet is a thin mask sheet.

8. The sheet mask as claimed in claim 1 or 2, wherein the mask sheet comprises 1 to 20 wt.% of an electrically conductive fiber containing a copper component, 1 to 20 wt.% of a non-conductive fiber containing a copper component, and 60 to 98 wt.% of a cotton fiber.

9. The sheet mask as claimed in claim 1 or 2, wherein the mask sheet comprises 1 to 20 wt.% of an electrically conductive fiber containing a copper component, 1 to 20 wt.% of a non-conductive fiber containing a copper component, 50 to 90 wt.% of a polyester fiber, and 1 to 20 wt.% of a spandex fiber.

10. The sheet mask as claimed in claim 1 or 2, wherein the mask sheet comprises 1 to 20 wt.% of an electrically conductive fiber containing a copper component, 1 to 20 wt.% of a non-conductive fiber containing a copper component, 10 to 40 wt.% of a cotton fiber, 10 to 30 wt.% of Tencel fiber, 20 to 50 wt.% of a polyester fiber, and 1 to 20 wt.% of a spandex fiber.

11. The sheet mask as claimed in claim 1 or 2, wherein the cosmetic composition comprises a copper component.

12. A knit fabric comprising 1 to 20 wt.% of an electrically conductive fiber containing a copper component.
